Europäisches Patentamt

European Patent Office

Office européen des brevets

(19)

(11) Veröffentlichungsnummer: **0 001 457**
**B1**

(12) **EUROPÄISCHE PATENTSCHRIFT**

(21) Anmeldenummer: **78200157.2**

(22) Anmeldetag: **28.08.78**

(51) Int. Cl.³: **C 07 C 9/14, C 07 C 7/08,// C 10 G 21/00**

(54) Verfahren zur Gewinnung von benzolfreiem n-Hexan

(30) Priorität: **11.10.77 DE 2745672**

(43) Veröffentlichungstag der Anmeldung:
**18.04.79 Patentblatt 79/08**

(45) Bekanntmachung des Hinweises auf die Patenterteilung:
**10.12.80 Patentblatt 80/25**

(84) Benannte Vertragsstaaten:
**BE DE FR GB NL**

(56) Entgegenhaltungen:
**DE - A - 2 359 300**

(73) Patentinhaber: **METALLGESELLSCHAFT AG**
**Reuterweg 14 Postfach 3724**
**D - 6000 Frankfurt M.1 (DE)**

(72) Erfinder: **Danulat, Hans-Friedrich, Dipl.-Ing.**
**Adalbert-Stifter-Strasse 4**
**D - 6000 Frankfurt am Main (DE)**
**John, Kamar Percy**
**Landwehrweg 51**
**D - 6380 Bad Homburg (DE)**
**Klein, Helmut, Dipl.-Ing.**
**Auf dem Unterfeld 2**
**D - 6450 Hanau (DE)**
**Lukatsch, Stephan, Dipl.-Ing.**
**Nordweststrasse 55**
**D - 6057 Dietzenbach (DE)**

(74) Vertreter: **Fischer, Ernst, Dr.**
**Reuterweg 14**
**D - 6000 Frankfurt am Main 1 (DE)**

Courier Press, Leamington Spa, England.

# 0 001 457

## Verfahren zur Gewinnung von benzolfreiem n-Hexan

Die Erfindung betrifft ein Verfahren zur Gewinnung von aromatenfreiem n-Hexan aus einem Gemisch von Benzol und Nichtaromaten durch Extraktivdestillation.

n-Hexan wird in der Industrie als Lösungsmittel und als Extraktionsmittel, beispielsweise bei der Extraktion von Ölsaat eingesetzt und gewinnt ständig an Bedeutung. Für viele Zwecke, insbesondere in der Nahrungsmittelindustrie, ist es erforderlich, daß das n-Hexan frei von Aromaten ist.

Zur Abtrennung von Aromaten aus Gemischen mit Nichtaromaten ist es bekannt, diese Gemische einer flüssig-flüssig-Extraktion oder einer Extraktivdestillation zu interwerfen, wobei diese Trennverfahren auch kombiniert werden können.

So ist beispielsweise ein Verfahren zur Gewinnung von Reinstaromaten aus aromatenhaltigen Kohlenwasserstoffgemischen durch Extraktion und Extraktivdestillation mit einem polaren Lösungsmittel und Aufarbeitung des von Nichtaromaten freien, aromatenhaltigen Extraktes durch Destillation bekannat, das dadurch gekennzeichnet ist, daß das gesamte aufzuarbeitende Gemisch einer Extraktivdestillation unterworfen wird, in der neben einem von Nichtaromaten freien Sumpfprodukt, das in an sich bekannter Weise durch Destillation auf Reinaromaten aufgearbeitet wird, ein Kopfprodukt abgenommen wird, das außer den gesamten Nichtaromaten 5 bis 30%, vorzugsweise 10 bis 20% der Aromaten des Einsatzgemisches enthält und in einer nachgeschalteten mehrstufigen flüssig-flüssig-Extraktion in ein nichtaromatisches Raffinat und einen die Aromaten des Kopfproduktes enthaltenden Extrakt zerlegt wird, der in den oberen Teil der Extraktivdestillationskolonne zurückgeführt wird (DT—PS 1 468 315).

Es ist ferner bekannt, zur Gewinnung reiner aromatischer Kohlenwasserstoffe aus ihren Gemischen mit nichtaromatischen Kohlenwasserstoffen durch Solventextraktion und/oder Extraktivdestillation mit einem selektiven, mit Abstand über den zu gewinnenden Aromaten siedenden Lösungsmittel, eine aus dem rohen Einsatzgemisch durch Vordestillation in ihrem Aromatengehalt angereicherte Einsatzfraktion der nachfolgenden Solventextraktion oder Extraktivdestillation aufzugeben, wobei der als Rücklauf auf die Vordestillation zurückgeführte Destillatanteil teilweise durch ein selektives und mit Abstand höher als die zu gewinnenden Aromaten siedendes wasserfreies Lösungsmittel ersetzt wird (DT—OS 2 122 770).

Ein weiteres Verfahren zur Gewinnung von reinen Aromaten aus Gemischen mit im gleichen Siedebereich siedenden Nichtaromaten mit Aromatengehalten von unter 60 Gew% durch Extraktivdestillation mit einem selektiven Lösungsmittel, ist dadurch gekennzeichnet, daß man das zur Abtrennung von Aromaten von den Nichtaromaten erforderliche Lösungsmittel zum Teil auf einen der oberen Böden einer.der eigentlichen Extraktivdestillationskolonne vorgeschalteten ersten Kolonne aufgibt, von dieser ein Kopfprodukt abzieht, vom Sumpf dieser Kolonne ein an Aromaten reiches Gemisch von Nichtaromaten, Lösungsmittel und Aromaten abzieht und dieses Sumpfprodukt durch extraktive Destillation in an sich bekannter Weise zu reinen Aromaten aufarbeitet (DE—OS 24 24 349).

Weiterhin ist ein Verfahren zur Trennung von n-Hexan aus seinen Benzolgemischen bekannt, wobei zwecks Gewinnung eines n-Hexan mit hohem Reinheitsgrad die Trennung in einem einzigen Rektifiziergang bei einem Druck zwischen 3 und 10 Atm für eine Säule mit einer Leistungsfähigkeit von etwa 40 theoretischen Böden durchgeführt wird, also unter Arbeitsbedingungen, unter denen sich das azeotrope, aus n-Hexan und Benzol bestehende Gemisch trennt. (DD—PS 31 301).

Bei diesen und ähnlichen Verfahren hängt die Menge der gewonnenen Aromaten von den Bedingungen beim Betrieb der Anlage ab. Bei Kokereibenzol beträgt normalerweise die Reinbenzolausbeute-bezogen auf das Einsatzprodukt—98 bis 99%. Bei Benzolschnitten, die aus Pyrolysebenzin oder Reformat gewonnen werden, beträgt der Gehalt an Reinbenzol im Raffinat ca. 3 bis 10%. Ein höherer Verlust an Reinbenzol im Raffinat würde die Betriebskosten der Anlage senken aber gleichzeitig die Ausbeute an Reinbenzol soweit vermindern, daß eine sinnvolle Gewinnung des Benzols nicht mehr gegeben ist. Eine Steigerung der Ausbeute ist aus energietechnischen Bedingungen meist nicht optimal und setzt die Wirtschaftlichkeit der Arbeitsweise herab.

Der Erfindung liegt die Aufgabe zugrunde, diese und andere Nachteile des Standes der Technik zu vermeiden und benzolfreies n-Hexan nach einem einfachen und wirtschaftlichen Verfahren zu gewinnen. Insbesondere soll das neue Verfahren energiesparend sein und die Gewinnung von n-Hexan, die bisher infolge der gleichzeitigen Anwsenheit von iso-Paraffinen und zyklischen Kohlenwasserstoffen neben Benzol sehr aufwendig und nur mit einer Feindestillation durchzuführen war, vereinfachen.

Diese Aufgabe wird erfindungsgemäß dadurch gelöst, daß man das aufzuarbeitende Gemisch in eine erste Destillierkolonne überführt, ein Benzol enthaltendes Sumpfprodukt und das aus leichtsiedenden Nichtaromaten bestehende Kopfprodukte abzieht, daß man das Destillat oberhalb der Zuführung des aufzuarbeitenden Gemischs seitlich abzieht und in den oberen Teil einer zweiten Destillierkolonne überführt, daß man aus dieser zweiten Destillierkolonne den Hexanschnitt, der aus Hexan, Benzol und geringen Mengen in dem Siedebereich der beiden Komponenten siedenden Nichtaromaten besteht, als Sumpfprodukt abzieht und etwà in der Mitte einer Extraktivdestillationskolonne aufgibt und hier mit einem selektiven Lösungsmittel extrahiert, das oberhalb der Zuführung des Hexanschnittes aufgegeben

2

**0 001 457**

wird, ein des selektive Lösungsmittel enthaltendes Sumpfprodukt abzieht und die über Kopf geführten Dämpfe kondensiert und als benzolfreies n-Hexan abzieht.

Nach einer Weiterbildung der Erfindung gibt man das aufzuarbeitende Gemisch aus Benzol und Nichtaromaten in etwa der Mitte der ersten Destillierkolonne auf.

Eine Ausgestaltung der Erfindung besteht darin, daß man das Kopfprodukt der ersten Destillation kondensiert und teilweise als Rückfluß auf dem Kopf der ersten Destillierkolonne aufgibt.

Nach einer bevorzugten Ausführungsform führt man einen Teil des kondensierten aus benzolfreiem n-Hexan bestehenden Kopfproduktes der Extraktivdestillation in den oberen Teil der Extraktivdestillation zurück.

Das Benzol und selektives Lösungsmittel enthaltende Sumpfprodukt der Extraktivdestillation kann man erfindungsgemäß in eine Stripperkolonne überführen und strippen, die Kopfdämpfe kondensieren und abziehen und teilweise in den oberen Teil der Stripperkolonne zurückführen.

Bevorzugt wird das in der Stripperkolonne als Sumpfprodukt erhaltene selektive Lösungsmittel in die Extraktivdestillation zurückgeführt.

Im Rahmen der Erfindung wird als selektives Lösungsmittel bevorzugt N-Methylpyrrolidon eingesetzt.

Anstelle der Stripperkolonne kann man erfindungsgemäß eine zur Gewinnung des aufzuarbeitenden Gemisches von Benzol und Nichtaromaten vorhandene Extraktivdestillationkolonne einsetzen.

Die mit der Erfindung erzielten Vorteile bestehen insbesondere darin, daß es auf eine einfache und wirtschaftliche Weise gelingt, benzolfreies n-Hexan aus Gemischen von Benzol und Nichtaromaten durch Extraktivdestillation herzustellen. Das neue Verahren ist sehr energiesparend.

Mit dem Verfahren ist es ferner möglich, alle Bestandteile des aufzuarbeitenden Gemisches auf umweltfreundliche Weise zu gewinnen.

Insbesondere gelingt die Gewinnung von Benzol in hoher Ausbeute und Reinheit.

Durch die niedere Temperatur, die bei der erfindungsgemäßen Hexan-Gewinnung angewandt wird, ist es möglich, die gesamte Destillation mit heißem Lösungsmittel weitgehend zu beheizen, und man benötigt nur eine geringe Menge an Niederdruckdampf oder Kondensat. Pro Tonne Hexan werden etwa 1,7 t 3 bar Dampf benötigt und etwa 17,9 t Kondensat, wobei das Kondensat von 133°C auf 100°C abgekühlt wird. Unter bar wird im Rahmen der Erfindung der Absolutdruck verstanden.

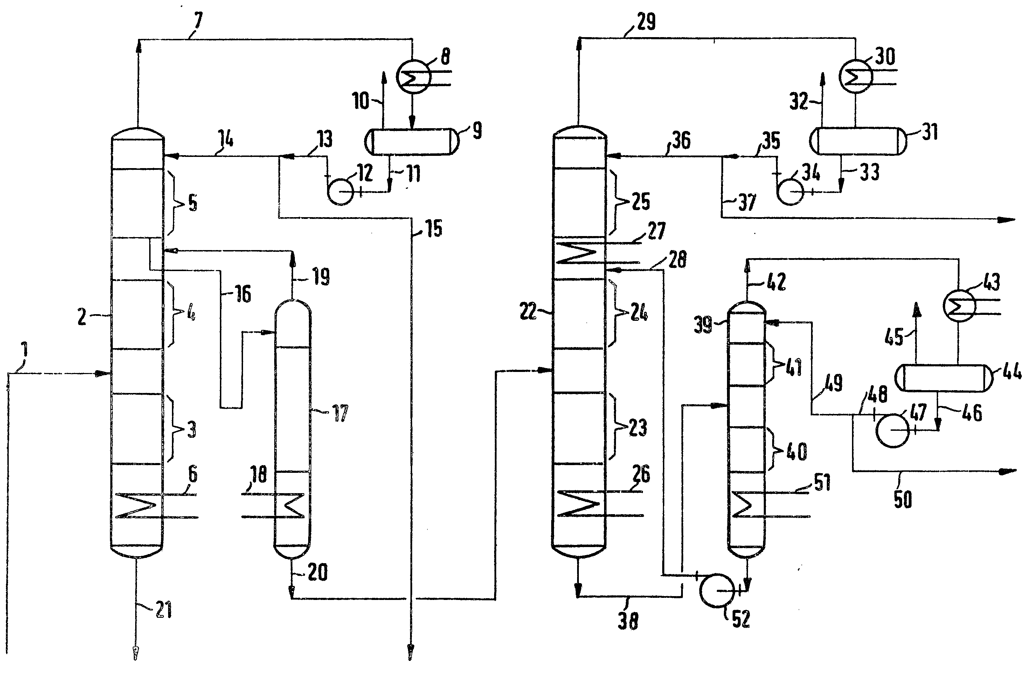

Die Erfindung wird anhand der Zeichnung und einem Ausführungsbeispiel im folgenden näher beschrieben und erläutert.

Ausführungsbeispiel

Über Leitung 1 wird das Kopfprodukt (Raffinat) einer Extraktivdestillations-Anlage (nicht dargestellt), bestehend aus

| | | | | | | |
|---|---|---|---|---|---|---|
| Cyclo-Pentan | 585 kg/h | $t_s = 49,3$ | °C | bei | 1 | bar |
| 3-Methyl-Pentan | 534 kg/h | $t_s = 63,3$ | " | " | " | " |
| n-Hexan | 1145 kg/h | $t_s = 68,7$ | " | " | " | " |
| Methyl-Pentan | 3777 kg/h | $t_s = 71,8$ | " | " | " | " |
| Benzol | 1086 kg/h | $t_s = 80,178$ | " | " | " | " |
| Cyclo-Hexan | 1403 kg/h | $t_s = 80,7$ | " | " | " | " |
| iso-Heptan | 1613 kg/h | $t_s = 90,0$ | " | " | " | " |
| Di-Methyl-Cyclo-Pentan | 1208 kg/h | $t_s = 90,8$ | " | " | " | " |
| n-Heptan | 1027 kg/h | $t_s = 98,4$ | " | " | " | " |
| Methyl-Cyclo-hexan | 149 kg/h | $t_s = 100,9$ | " | " | " | " |
| | 12527 kg/h | | | | | |

einer Vordestillationskolonne 2 zugeführt. Das Sumpfprodukt dieser Kolonne wird über Leitung 21 abgezogen und hat folgende Zusammensetzung:

3

| | |
|---|---|
| 3-Methyl-Pentan | 2 kg/h |
| n-Hexan | 95 kg/h |
| Methyl-Pentan | 1300 kg/h |
| Benzol | 350 kg/h |
| Cyclo-Hexan | 1053 kg/h |
| iso-Heptan | 1605 kg/h |
| Di-Methyl-Cyclo-Pentan | 1208 kg/h |
| n-Heptan | 1027 kg/h |
| Methyl-Cyclo-Hexan | 149 kg/h |

Die Destillationswärme wird mit Heizregister 6 der Kolonne zugeführt. In der Kolonne sind Kolonnenböden angeordnet, die zweckmäßigerweise zu den Paketen 3, 4 und 5 zusammengefaßt sind. Zwischen Paket 3 und 4 erfolgt die Aufgabe des Raffinates über Leitung 1. Über Leitung 7 werden die Kopfdämpfe abgezogen, im Kondensator 8 niedergeschlagen und im Rückflußbehälter 9 gesammelt. Die Druckhaltung der Kolonne erfolgt über Leitung 10. Über Leitung 11 wird mit Pumpe 12 und Leitung 13 das verflüssigte Kopfprodukt gefördert, wobei über Leitung 14 ein Teil als Rücklauf aufgegeben wird und über Leitung 15 leichtsiedende Nichtaromaten als Fertigprodukt gewonnen werden. Sie haben folgende Zusammensetzung:

| | |
|---|---|
| Cyclo-Pentan | 556 kg/h |
| 3-Methyl-Pentan | 282 kg/h |
| n-Hexan | 40 kg/h |
| | 878 kg/h |

Im unteren Teil des Bodenpaketes 5 wird ein Seitenprodukt über Leitung 16 flüssig entnommen, das in die zweite Kolonne 17 geführt wird. Diese Kolonne enthält nur ein einziges Bodenpaket, bestehend aus ca. 40 Austauschböden. Die Destillationswärme wird über Heizregister 18 der Kolonne 17 zugeführt. Die Kopfdämpfe der Kolonne 17 werden über Leitung 19 in die Kolonne 2 zurückgeführt. Das Sumpfprodukt der Kolonne 17 wird über Leitung 20 der Extraktivdestillationskolonne 22 aufgegeben. Das Produkt in der Leitung 20 ist die Hexan-Fraktion, die folgende Zusammensetzung hat:

| | |
|---|---|
| Cyclo-Pentan | 29 kg/h |
| 3-Methyl-Pentan | 250 kg/h |
| n-Hexan | 1010 kg/h |
| Methyl-Pentan | 2477 kg/h |
| Benzol | 736 kg/h |
| Cyclo-Hexan | 350 kg/h |
| iso-Heptan | 8 kg/h |
| | 4860 kg/h |

Die Extraktivdestillationskolonne 22 besteht aus dem Heizsystem 26, den zu Paketen vereinigten Böden 23, 24, 25 und einem Zwischenaufkocher 27. Auf die Böden des Bodenpaketes 24 wird über

4

Leitung 28 N-Methylpyrrolidon als Lösungsmittel aufgegeben. Die Menge dieses Lösungsmittels beträgt 20.000 kg/h. Über Leitung 38 wird das Sumpfprodukt, bestehend aus

| | |
|---|---|
| Cyclo-Pentan | 4 kg/h |
| 3-Methyl-Pentan | 40 kg/h |
| n-Hexan | 5 kg/h |
| Methyl-Pentan | 2377 kg/h |
| Benzol | 736 kg/h |
| Cyclo-Hexan | 350 kg/h |
| iso-Heptan | 8 kg/h |
| N-Methylpyrrolidon | 20000 kg/h |
| | 23520 kg/h |

abgezogen und entweder der vorgeschalteten Extraktivdestillation (nicht dargestellt) zugeführt oder einer nachgeschalteten Stripperkolonne 39 aufgegeben. Das Kopfprodukt der Kolonne 22 wird über Leitung 29 dampfförmig in den Kondensator 30 geführt, dort verflüssigt und fließt im Rückflußbehälter 31 ab. Die Druckhaltung der Kolonne erfolgt über Leitung 32. Über Leitung 33, Pumpe 34 und Leitung 35 wird das verflüssigte Kopfprodukt abgezogen und teilweise als Rücklauf mit Leistung 36 aufgegeben, teilweise über Leitung 37 als aromatenfreies Endprodukt abgezogen. Dieses Produkt ist das n-Hexan, welches mit folgender Zusammensetzung anfällt:

| | |
|---|---|
| Cyclo-Pentan | 25 kg/h |
| 3-Methyl-Pentan | 210 kg/h |
| n-Hexan | 1005 kg/h |
| Methyl-Pentan | 100 kg/h |
| | 1340 kg/h |

Die Abtrennung des Benzols und der unerwünschten Nichtaromaten aus dem Hexan-Schnitt, welcher über Leitung 20 der Extraktivdestillationskolonne 22 zugeführt wird, erfolgt unterhalb der Aufgabe des N-Methylpyrrolidons in den Bodenpaketen 24 und 23. Mit Heizregister 27 und in dem Bodenpaket 25 erfolgt, unter Einfluß des Rückflusses über Leitung 36, die Reinigung des Hexans von Spuren von mitaufsteigendem N-Methylpyrrolidon.

In der Stripperkolonne 39, die aus dem Heizregister 51 und den zu Paketen 40 und 41 angeordneten Böden besteht, wird als Sumfprodukt mit Pumpe 52 über Leitung 28 das N-Methylpyrrolidon abgezogen und auf die Extraktivdestillationskolonne 22 aufgegeben. Über Leitung 42 werden die Kopfdämpfe abgezogen und im Kondensator 43 verflüssigt und im Rückflußbehälter 44 gesammelt. Die Druckhaltung erfolgt über Leitung 45. Über Leitung 46 wird mit Pumpe 47 und Leitung 48 das Kopfprodukt einerseits als Rücklauf über Leitung 49 der Kolonne 39 aufgegeben, andererseits als Produkt über Leitung 50 abgezogen. Dieses Produkt hat folgende Zusammensetzung:

| | |
|---|---|
| Cyclo-Pentan | 4 kg/h |
| 3-Methyl-Pentan | 40 kg/h |
| n-Hexan | 5 kg/h |
| Methyl-Pentan | 2377 kg/h |
| Benzol | 736 kg/h |
| Cyclo-Hexan | 350 kg/h |
| iso-Heptan | 8 kg/h |
| | 3520 kg/h |

Die über Leitung 21 abgezogenen Nichtaromaten sind in diesem Falle noch benzolhaltig. Die über Leitung 15 abgezogenen Nichtaromaten sind frei von Benzol. Das über Leitung 37 abgezogene Hexan ist aromaten- und lösungsmittelfrei. Das über Leitung 50 abgezogene Benzol enthält noch Nichtaromaten und ist lösungsmittelfrei.

Benützt man statt der Kolonne 39 die vorhandene zur Benzolgewinnung vorgeschaltete Extraktivdestillationsanlage, so wird Leitung 38 in die Zuflußleitung zu dieser Extraktivdestillationsanlage geführt, d.h. in die Zuflußleitung zur Extraktivdestillationskolonne eingebunden. Das Lösungsmittel, das über Leitung 28 der Extraktivdestillationskolonne 22 zugeführt wird, wird in diesem Falle als Teilstrom aus dem Sumpf der vorhandenen Stripperkolonne genommen. Im Kopf der Stripperkolonne fällt die im Raffinat der Extraktivdestillation (Einsatz in Leitung 1) zusätzlich mitgewonnene Menge Benzol an, wodurch die Benzolausbeute dort etwa 100% beträgt.

Das Vordestillations-Kolonnensystem, bestehend aus Kolonne 2 und Kolonne 17, arbeitet bei einem Kopfdruck von 1,3 bar. Dadurch entstehen Sumpftemperaturen von 80 bis 90°C. Die Extraktivdestillationskolonne 22 arbeitet unter einem Kopfdruck von 0,8 bar. Die Sumpftemperatur dieser Kolonne beträgt ca. 108°C. Die Stripperkolonne arbeitet mit einer Sumpftemperatur von 165°C bei einem Druck von 0,5 bar. In der Figur ist nicht dargestellt, welche Wärmeaustauschmöglichkeiten bestehen. Aus dem Vorhergesagten ergibt sich, daß fast die gesamte Wärme des Lösungsmittels bei einer Temperaturabsenkung von 165°C auf 60°C zur Beheizung der Destillation verwendet werden kann. Es ist lediglich Wärme aufzubringen für das Heizregister der Extraktivdestillationskolonne 26. Zur Beheizung dienen 3 bar Dampf, im vorliegenden Falle 1700 kg/h. Mit dem Kondensat aus diesem Dampf und Fremdkondensat wird die Kolonne 2 beheizt. Es werden an Kondensat 19600 kg/h benötigt. Ist eine Extraktivdestillationsanlage zur Benzolgewinnung vorhanden, dann wird von dort eine Wärmemenge von 1.500.000 kcal/h für die Beheizung der Anlage bereitgestellt. Ist eine separate Stripperkolonne nötig, dann muß diese Wärmemenge mit 10 bis 12 bar Dampf zugeführt werden. Die im Beispiel genannten Werte gelten für eine Ausbeute an n-Hexan von 90%, bezogen auf die n-Hexan-Menge in Leitung 1 = 100%.

| | |
|---|---|
| Das eingesetzte Raffinat hat einen Preis von ca. | DM 4 900,-/h |
| und der benützte Dampf | DM 43,-/h |
| das Fremdkondensat ca. | DM 40,-/h |
| Total | DM 4 938,-/h |

Der Gewinn = DM 557 -/h.

Dieser Gewinn gilt, wenn die Hexan-Gewinnung/Entaromatisierung einer vorhandenen Extraktivdestillationsanlage nachgeschaltet ist. Muß ein separater Stripper betrieben werden, dann werden zusätz-lich 2,3 t Dampf pro Stunde benötigt und die Verbrauchskosten steigen um DM 57,-, was den Gewinn um DM 57,- reduziert, d.h. auf DM 500,-/h.

**Patentansprüche:**

1. Verfahren zur Gewinnung von benzolfreiem n-Hexan aus einem dieses, Benzol und andere Nichtaromaten enthaltenden Gemisch durch Extraktivdestillation, dadurch gekennzeichnet, daß man das aufzuarbeitende Gemisch in eine erste Destillierkolonne überführt, ein Benzol enthaltendes Sumpfprodukt und das aus leichtsiedenden Nichtaromaten bestehende Kopfprodukt abzieht, daß man das Destillat oberhalb der Zuführung des aufzuarbeitenden Gemischs seitlich abzieht und in den oberen Teil einer zweiten Destillierkolonne überführt, daß man aus dieser zweiten Destillierkolonne den Hexanschnitt, der aus Hexan, Benzol und geringen Mengen in dem Siedebereich der beiden Komponenten siedenden Nichtaromaten besteht, als Sumpfprodukt abzieht und etwa in der Mitte einer Extraktivdestillationskolonne aufgibt und hier mit einem selektiven Lösungsmittel extrahiert, das oberhalb der Zuführung des Hexanschnittes aufgegeben wird, ein das selektive Lösungsmittel enthaltendes Sumpfprodukt abzieht und die über Kopf geführten Dämpfe kondensiert und als benzolfreies n-Hexan abzieht.

2. Verfahren nach Anspruch 1, dadurch gekennzeichnet, daß man das aufzuarbeitende Gemisch in etwa der Mitte der ersten Destillierkolonne aufgibt.

3. Verfahren nach Anspruch 1 und 2, dadurch gekennzeichnet, daß man das Kopfprodukt der ersten Destillation kondensiert und teilweise als Rückfluß auf dem Kopf der ersten Destillierkolonne aufgibt.

4. Verfahren nach Anspruch 1 bis 3, dadurch gekennzeichnet, daß man einen Teil des kondensierten aus benzolfreiem n-Hexan bestehenden Kopfproduktes der Extraktivdestillation in den oberen Teil der Extraktivdestillation zurückführt.

5. Verfahren nach einem oder mehreren der vorhergehenden Ansprüche 1 bis 4, dadurch gekennzeichnet, daß man das Benzol und ein selektives Lösungsmittel enthaltendes Sumpfprodukt der Extraktivdestillation in eine Stripperkolonne überführt und strippt, die Kopfdämpfe kondensiert und abzieht und teilweise in den oberen Teil der Stripperkolonne zurückführt.

6. Verfahren nach einem oder mehreren der vorhergehenden Ansprüche 1 bis 5, dadurch gekennzeichnet, daß man das in der Stripperkolonne als Sumpfprodukt erhaltene selektive Lösungsmittel in die Extraktivdestillation zurückführt.

7. Verfahren nach einem oder mehreren der vorhergehenden Ansprüche 1 bis 6, dadurch gekennzeichnet, daß man als selektives Lösungsmittel N-Methylpyrroliden einsetzt.

8. Verfahren nach einem oder mehreren der vorhergehenden Ansprüche 1 bis 7, dadurch gekennzeichnet, daß man anstelle der Stripperkolonne eine Extraktivdestillationskolonne einsetzt.

**Revendications**

1. Procédé pour obtenir de l'hexane normal exempt de benzène à partir d'un mélange en contenant ainsi que des produits aromatiques et d'autres produits non aromatiques par distillation extractive, caractérisé en ce qu'il consiste à envoyer le mélange à traiter dans une première colonne de distillation, à soutirer un produit de queue contenant du benzène et un produit de tête constitué des produits non aromatiques à bas point d'ébullition, à soutirer latéralement le distillat au-dessus de l'amenée du mélange à traiter et à envoyer le distillat à la partie supérieure d'une deuxième colonne de distillation, à soutirer à titre de produit de queue de la deuxième colonne de distillation la coupe hexanique qui se compose d'hexane, de benzène et de faibles quantités de produits non aromatiques bouillant dans la gamme d'ébullition des deux constituants précités et à charger cette coupe hexanique à peu près au milieu d'une colonne de distillation extractive et à extraire à l'aide d'un solvant sélectif qui est chargé au-dessus de l'amenée de la coupe hexanique, à soutirer un produit de queue contenant le solvant sélectif, à condenser les vapeurs de tête et à soutirer de l'hexane normal exempt de benzène.

2. Procédé suivant la revendication 1, caractérisé en ce qu'il consiste à charger le mélange à traiter à peu près au milieu de la première colonne de distillation.

3. Procédé suivant la revendication 1 ou 2, caractérisé en ce qu'il consiste à condenser le produit de tête de la première distillation et à le charger partiellement à titre de reflux en tête de la première colonne de distillation.

4. Procédé suivant la revendication 1 à 3, caractérisé en ce qu'il consiste à retourner à la partie supérieure de la distillation extractive une partie du produit de tête condensé de la distillation extractive constitué d'hexane normal exempt de benzène.

5. Procédé suivant l'une des revendications 1 à 4, caractérisé en ce qu'il consiste à envoyer dans une colonne de rectification le benzène et un produit de queue de la distillation extractive contenant un solvant sélectif et à y effectuer une rectification, à condenser les vapeurs de tête et à les soutirer et à les retourner partiellement à la partie supérieure de la colonne de rectification.

6. Procédé suivant l'une des revendications 1 à 5, caractérisé en ce qu'il consiste à retourner à la distillation extractive le solvant sélectif obtenu dans la colonne de rectification à titre de produit de queue.

7. Procédé suivant l'une des revendications précédentes, caractérisé en ce qu'il consiste à utiliser comme solvant sélectif la N-méthylpyrrolidone.

8. Procédé suivant l'une des revendications 1 à 7, caractérisé en ce qu'il consiste à utiliser au lieu d'une colonne de rectification une colonne de distillation extractive.

**0 001 457**

Claims

1. A process of recovering an n-hexane product which is free from benzene and non- aromatic components by extractive distillation, from a mixture of n-hexane, aromatic and non-aromatic compounds, characterized in that a feed mixture is fed to a first distillation column and distilled therein, a benzene-containing sump product and an overhead product consisting of the low-boiling non-aromatic compounds are withdrawn from the first distillation column, and a distillate is laterally withdrawn from the first distillation column at a point disposed above the feeding point of the feed mixture and is transferred to the upper portion of a second distillation column, a sump product consisting of the hexane cut consisting of hexane, benzene and small amounts of non-aromatics boiling in the range of both of said components is withdrawn from the second distillation column and is fed to an extractive distillation column approximately in the middle thereof and extracted therein with a selective solvent, which is fed to the extractive distillation column above the feeding point of the hexane cut a sump product containing the selective solvent is withdrawn from the extractive distillation column, vapors are withdrawn overhead from the extractive distillation column and condensed, and the condensate is withdrawn as an n-hexane product which is free from benzene.

2. A process according to claim 1, characterized in that the feed mixture is fed to the first distillation column approximately in the middle thereof.

3. A process according to claim 1 and 2, characterized in that the overhead product of the first distillation column is condensed and is partly refluxed to the top of the first distillation column.

4. A process according to claim 1 to 3, characterized in that part of the condensed overhead product of the extractive distillation, consisting of an n-hexane product which is free from benzene, is recycled to the upper portion of the extractive distillation column.

5. A process according to any of the preceding claims 1 to 4, characterized in that the sump product of the extractive distillation column, which sump product contains benzene and a selective solvent, is transferred to a stripping column and stripped therein, the overhead vapors of the stripping column are condensed, and the condensate is withdrawn and partly recycled to the upper portion of the stripping column.

6. A process according to any of the preceding claims 1 to 5, characterized in that the selective solvent which becomes available in the stripping column as a sump product is recycled to the extractive distillation column.

7. A process according to any of the preceding claims 1 to 6, characterized in the N-methylpyrrolidone is used as a selective solvent.

8. A process according to any of the preceding claims 1 to 7, characterized in that an extractive distillation column is used instead of the stripping column.

8

0 001 457